# EUROPEAN PATENT APPLICATION

(11) **EP 4 282 330 A1**
(43) Date of publication of application: **29.11.2023**
(21) Application number: 22382500.1
(22) Date of filing: 25.05.2022
(51) Int. Cl.: A61B 5/103, A61B 5/00, G16H 50/20, G01J 3/52

(54) **AI MARKER DEVICE, METHOD FOR STANDARDISING AN IMAGE USING THE AI MARKER DEVICE AND METHOD FOR GRADING THE SEVERITY OF A SKIN DISEASE USING BOTH**

(71) Applicant: AI Labs Group, S.L., 48013 Bilbao Vizcaya (ES)
(72) Inventor: MAC CARTHY ESPINAR, Taig, 48013 BILBAO (Vizcaya) (ES); MEDELA AYARZAGUENA, Alfonso, 48013 BILBAO (Vizcaya) (ES)
(74) Representative: IP HILLS NV

(57) **Abstract**

The invention relates to an Ai marker device, a method for standardising an image using the Ai marker device and a method for grading the severity of a skin disease using both. The Ai marker device comprises a colour roulette (2) configured to standardise hue, brightness, saturation, dimension and perspective of an image, a grayscale strip (3) configured to standardise brightness of the image, four warping elements (4) configured to correct a perspective distortion of the image, a Siemens star (5) configured to calculate resolution of the image, and a registration mark (6) configured to detect the Ai marker at first sight. The method for standardising an image generates a standardised image in size, colour and perspective. The method for grading the severity of a skin disease is based on comparisons between standardised images.

## Description

### Object of the invention

The invention consists of an Ai marker device, a method for standardising an image using the Ai marker device and a method for grading the severity of a skin disease using both in combination.

An object of the invention is to provide a marker device that enables the application of Artificial Intelligence algorithms.

Another object of the invention is to provide a method for standardising an image, wherein the Ai marker device is contained in the image taken. The method allows to compare different images regardless of the perspective, tone, hue and saturation.

Another object of the invention is to provide a method for grading the severity of a skin disease. The method for grading the severity of a skin disease is based on the comparison between standardised images in which the Ai Marker device is contained, and using the method for standardising an image.

### Technical problem to be solved and background of the invention

In the state of the art, it is known the PCT patent application with publication number WO 2010/093503 A2, which relates to determining a skin photo type of a captured image in a Red Green Blue (RGB) colour imaging system and is also applicable in classification of other skin characteristics (e.g. elasticity, melanin, oil concentration etc.), melanoma, skin related tumours and skin related disorders. WO 2010/093503A2 discloses a skin care device that includes an electromagnetic radiation source capable of directing incident electromagnetic radiation to a location on the skin of a user, a radiation detector for measuring various parameters of radiation re-emitted from the location, and a skin condition analysis module coupled to the detector, the analysis module capable of generating a skin condition assessment in real-time, based partly on at least one of RGB analysis and diffused reflectance analysis of the radiation parameters (visible, near-infrared, and near-ultraviolet spectrum).

There is a need for a minimal error and speed-efficient method and device to determine the severity of skin diseases without using electromagnetic radiation devices which may be contraindicated. Likewise, there is a need for a method and device that is minimally invasive in its interaction with the tissue so as to not affect the disease. Furthermore, there is a need for a method and device that patients can use from their homes without the intervention of a specialist, in order to facilitate remote care situations and telemedicine processes. For this to be possible, the method and device must be easy to operate, the method must require the minimum maintenance and it must be affordable enough for the average medicine cabinet.

### Description of the invention

According to a first aspect of the invention there is provided an Artificial Intelligence "Ai" marker device for standardising an image. The Ai marker device for standardising an image comprises:
- a colour roulette configured to standardise hue, brightness, saturation, dimension and perspective of an image; the colour roulette comprising:
   ∘ a bottom semi-circumference comprising at least six colours with specific pigment values, wherein the six colours having three CMY pure pigment values and three CMY pure pigment values at 50%;
- a grayscale strip under the colour roulette configured to standardise brightness of the image, the grayscale strip comprises a gradient of tints of black;
- four warping elements forming vertices of a square in which the colour roulette is circumscribed, the four warping elements configured to correct a perspective of the image;
- a Siemens star placed between the grayscale strip and the colour roulette configured to calculate resolution of the image;
- a registration mark configured to detect the Ai marker at first sight.
The colour roulette, the grayscale strip, the four warping elements, the Siemens star and the registration mark are placed on a transparent surface, which is cutout inside the colour roulette. That is, the surface material in the inner circle inscribed inside the colour roulette is cut-out.

In an arrangement, the bottom semi-circumference further comprises three additional colours having three RGB pure pigment values and three more colours that are exact combinations of the RGB pure pigment values.

In an arrangement, the colour roulette further comprises a top semi-circumference consisting of twelve skin-like colours with specific pigment values that match different skin phototypes.

In an arrangement, the transparent surface may be manufactured in a transparent plastic film and laminate polyethylene "PE" transparent paper, or it may be manufactured in polypropylene "PP".

In an arrangement, the top semi-circumference of the colour roulette may have the following twelve different pigment values, i.e. colours, in CMYK scale, placed clockwise, that accurately represent skin-like colours with specific pigment values that match different skin phototypes:

| C | M | Y | K |
|---|---|---|---|
| 0% | 20% | 10% | 0% |
| 0% | 30% | 20% | 0% |
| 0% | 30% | 30% | 0% |
| 0% | 20% | 30% | 0% |
| 0% | 20% | 30% | 10% |
| 0% | 30% | 20% | 20% |
| 0% | 20% | 30% | 20% |
| 0% | 20% | 30% | 30% |
| 0% | 30% | 30% | 50% |
| 0% | 20% | 30% | 50% |
| 0% | 40% | 40% | 50% |
| 0% | 40% | 40% | 70% |

In an arrangement, the bottom semi-circumference may have the following twelve different pigment values, i.e. colours, that match specific values in both the CMYK and the RGB colour spaces, placed counterclockwise:

| C | M | Y | K |
|---|---|---|---|
| 0% | 100% | 100% | 0% |
| 0% | 50% | 100% | 0% |
| 100% | 0% | 100% | 0% |
| 50% | 0% | 100% | 0% |
| 100% | 100% | 0% | 0% |
| 50% | 100% | 0% | 0% |
| 100% | 0% | 0% | 0% |
| 50% | 0% | 0% | 0% |
| 0% | 100% | 0% | 0% |
| 0% | 50% | 0% | 0% |
| 0% | 0% | 100% | 0% |
| 0% | 0% | 50% | 0% |

Each colour of the colour roulette is separated by white stripes. The colour roulette is externally and internally surrounded by white circumferences. The circle inside the internal circumference is cut-out. Outside the external circumference, the surface is transparent.

In an arrangement, the grayscale strip may comprise seven squares: a first square, a second square, a third square, a fourth square, a fifth square, a sixth square and a seventh square, placed in a row and separated by stripes. The first square is black and the seventh square is white. The second square, the third square, the fourth square, the fifth square and the sixth square are tints of grey that range from dark to light grey. The stripes between the first square to the fifth square are white and the stripes between the fifth square to the seventh square are black. Specifically, the squares have the following CMYK values:

| Square | C | M | Y | K |
|---|---|---|---|---|
| 1st | 0% | 0% | 0% | 100% |
| 2nd | 0% | 0% | 0% | 90% |
| 3rd | 0% | 0% | 0% | 80% |
| 4th | 0% | 0% | 0% | 60% |
| 5th | 0% | 0% | 0% | 40% |
| 6th | 0% | 0% | 0% | 20% |
| 7th | 0% | 0% | 0% | 0% |

In an arrangement, the transparent surface may be in the shape of a rectangle. Thus, the whole Ai marker device may be in the shape of a rectangle. The edges of the rectangle are two of the four warping elements and the first and seventh squares of the grayscalestrip.

In an arrangement, each warping element may comprise a black square with a white square inside, placed parallel one to each other. Each warping element may comprise a white "L" bracket externally attached to two sides of the black square.

In an arrangement, the Siemens star may comprise a pattern of bright spokes on a dark background that radiate from a common centre and become wider as the bright spokes get further from the centre.

In an arrangement, the registration mark may be a black and white target icon having a centred cross.

According to a second aspect of the invention there is provided a computer-implemented method for standardising an image using an Ai marker device according to the first aspect of the invention. The computer-implemented method for standardising an image using an Ai marker device comprises the following steps:
a) starting the Ai Marker detection process, comprising:
   ∘ locating an Ai marker device in an image by finding the colour roulette, the four warping elements, the grayscale strip, the Siemens star and the registration mark of the Ai marker device; if the Ai marker device is not found, prompt the user to retake the image; if it is found, continue;
   ∘ calculating four coordinates of pixels of the image in which edges of the Ai Marker device are placed;
b) starting the colour standardisation process, comprising:
   ∘ detecting pigment values of the colour roulette and the grayscale strip in the image;
   ∘ creating a mask of vectors that contains the location and shape of each colour of the roulette and the grayscale strip; and,
   ∘ performing a transformation matrix to standardise the hue and saturation, by matching the colour values as they are captured in the image against the known pigment values of the colour roulette; (colour is standardised)
c) starting the perspective standardisation process, comprising:
   ∘ detecting the four warping elements;
   ∘ adding the location and shape of each warping element to the vector mask;
   ∘ obtaining the coordinates of the contour and the coordinates of the edges of the four warping elements and the grayscale strip;
   ∘ correcting the perspective by means warping the image so that the plane is perfectly perpendicular; and,
   ∘ correcting the perspective by means of rotating the image; (perspective is standardised)
d) starting the size standardisation process, comprising:
   ∘ calculating the area of the warping elements in square pixels by counting the number of pixels that conform the warping elements in the vector mask;
   ∘ converting the pixels of each warping element to metric units values matching the area in square pixels against the known dimensions in square millimetres;
   ∘ performing a 2D-interpolation and 2D-extrapolation to calculate the value in metric units of each and every single pixel of the image; and,
      (size is standardised)
   ∘ generating a standardised image in size, colour and perspective.

In an arrangement, the method may further comprise starting the resolution estimation process, comprising:
∘ looking for a Siemens star;
∘ calculating resolution and information about the resolution by:
   ▪ counting how many pixels constitute the spikes of the Siemens star;
   ▪ comparing the count against the known dimensions;
   ▪ returning a value that reflects the resolution of the image.

The output of the computer-implemented method for standardising an image is a standardised image in which size, colour and perspective have been corrected.

The detected pigment values of the colour roulette in the image may be the six up to the twelve pigment values of the bottom semi-circumference. Furthermore, the detected pigment values of the colour roulette may be the six up to the twelve pigment values of the bottom semi-circumference combined with the twelve pigment values of the top semi-circumference.

According to a third aspect of the invention there is provided a method for grading the severity of a skin disease, wherein the method comprises the steps of:
a) providing an Ai marker device according to the first aspect of the invention;
b) placing the Ai marker device in the vicinity of a skin disease of a patient and taking an image of the skin with the Ai marker device;
c) applying a computer-implemented method for standardising an image according to the second aspect of the invention, that returns the standardised image;
d) starting the scoring of the intensity of the clinical signs, comprising:
   ∘ for each clinical sign, running a multi-output classification convolutional neural network that returns the intensity of each clinical sign;
   ∘ normalising and rounding to a range the intensity of the clinical signs that returns the score of the clinical signs;
   ∘ aggregating all the scores;
e) starting the scoring of the affected surface, comprising:
   ∘ running a segmentational convolutional neural network, that returns a vector mask of the affected surface;
   ∘ retrieving the standardised image that was calculated on step c) and enables the standardisation of the size of the image;
   ∘ calculating the surface of the lesion contained in the image relative to the whole body surface area;
   ∘ categorising the percentage of the affected body area into ranges, which constitutes the score of the affected surface area.

In an arrangement, the method may further comprise:
f) if the user is taking more than one image of the same patient, running previous steps a) to e) and applying them to further pictures/images, which return the scores of the intensity of the clinical signs and the score of the affected surface area for each image;
g) retrieving the scoring system's formula in a database of medical scoring systems;
h) applying the scoring system's formula to the scores of the intensity of the clinical signs and the score of the affected surface area, that returns a final score that reflects the severity of the disease; and,
i) displaying the final score.

### Description of the figures

To further clarify the description, and for the purpose of helping to make the features of the invention more readily understandable, this specification is accompanied by a set of drawings that constitute an integral part of the specification, wherein, by way of illustration and not limitation, the following has been represented:
Figures 1A and 1B show the artificial intelligence marker device of the present invention. For purposes of representing the difference between the white colour and the transparent surface, figure 1B is shown with a light-grey background that is not part of the device. Likewise, for purposes of representing the difference between the transparent surface and the cut-out hole inside the colour roulette, figure 1B shows a dark-grey background inside the colour roulette that is not part of the device.
Figure 2 shows the artificial intelligence marker device of the present invention depicting the value in CMYK scale of each pigment value.
Figure 3 shows the top semi-circumference and the bottom semi-circumference of the colour roulette.
Figure 4 shows the grayscale strip in detail. For purposes of representing the difference between the white colour and the transparent surface, figure 4 shows a discontinuous stroke around the white area that is not part of the device.
Figure 5 shows one of the warping elements in detail. For purposes of representing the difference between the white colour and the transparent surface, figure 4 shows a discontinuous stroke around the white area that is not part of the device.
Figure 6 shows the Siemens star in detail.
Figure 7 shows the registration mark in detail.
Figure 8 shows a flow chart showing the steps for taking an image of a disease.
Figures 9A, 9B, 9C and 9D show flow charts depicting the steps from detecting the Ai Marker device in the taken image to obtaining a standardised image with resolution information using the computer-implemented method for standardising an image and the Ai marker device.
Figures 10A, 10B, 10C, 10D show flow charts depicting the steps of a method for grading the severity of a skin disease using the Ai marker device and the computer-implemented method for standardising an image of the present invention.

### Preferred embodiment of the invention

The invention is described below based on the aforementioned figures.

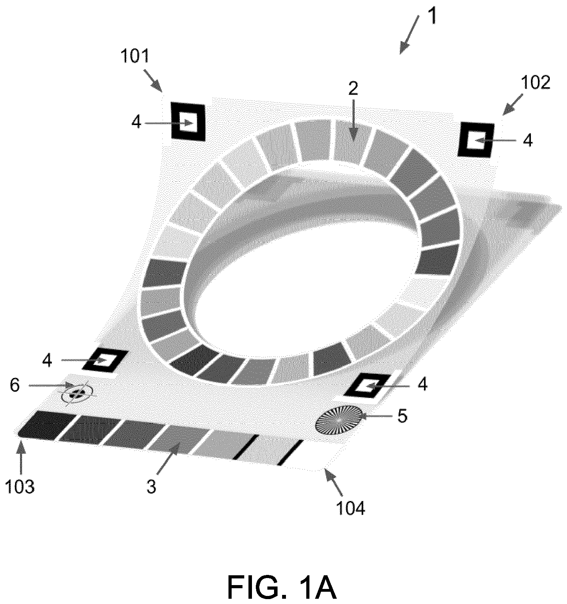

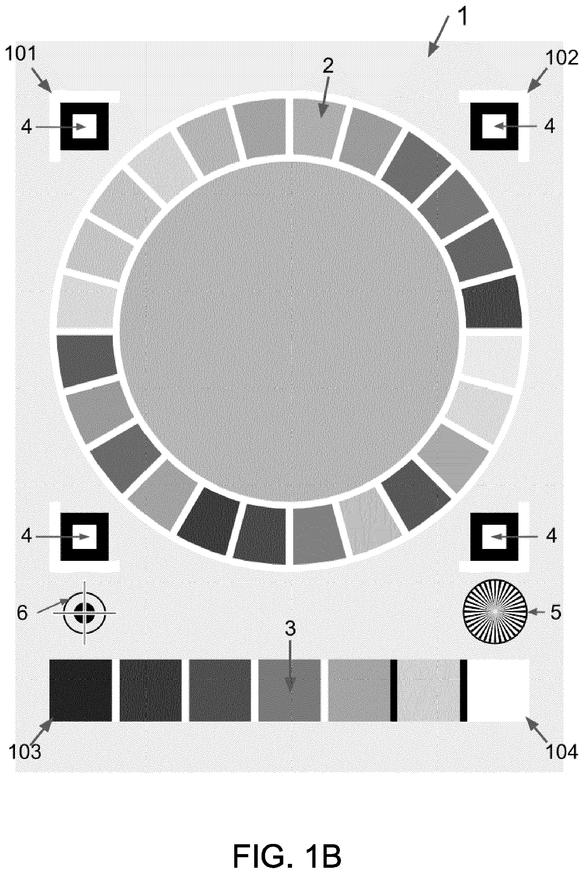

Figures 1A and 1B show the Artificial Intelligence "Ai" marker device 1 of the present invention. Since the device is manufactured over a transparent surface, and it also has white areas. To make the figures understandable, Figure 1B shows the transparent surface represented by a light-grey colour that is different from any of the grey colours of the grayscale strip 3. The Ai marker device 1 shown in Figures 1A and 1B comprises the colour roulette 2, the grayscale strip 3 below the colour roulette 2, the four warping elements 4, the Siemens star 5 and the registration mark 6. The colour roulette 2, the grayscale strip 3, the four warping elements 4, the Siemens star 5 and the registration mark 6 are placed on a transparent surface. The transparent surface inside the colour roulette 2 is cut-out, which is depicted in a dark-grey colour that is different from any of the grey colours of the grayscale strip 3 and also from the grey colour that represents the transparent surface.

The transparent surface can be manufactured in any kind of transparent material such as a transparent plastic film and laminate polyethylene (PE) transparent paper, or be manufactured in polypropylene (PP).

The Siemens star 5 is placed between the grayscale strip 3 and the colour roulette 2, under the bottom right warping element 4. The registration mark 6 is placed between the grayscale strip 3 and the colour roulette 2, under the bottom left warping element 4.

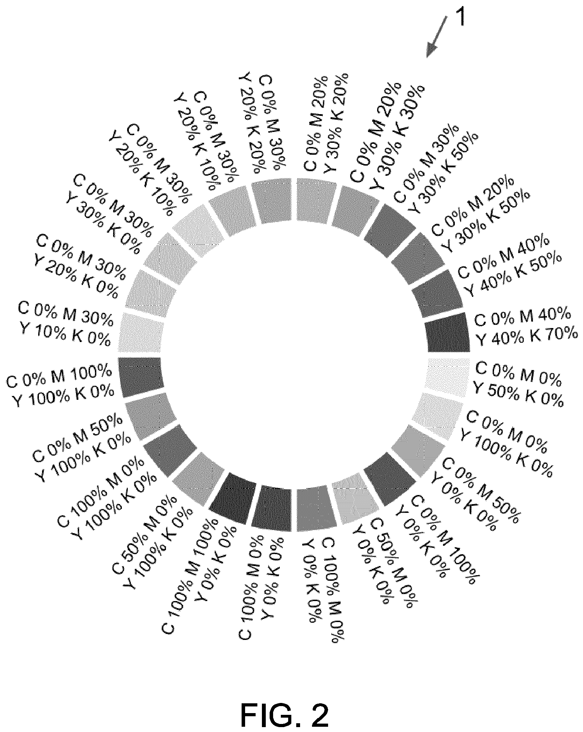

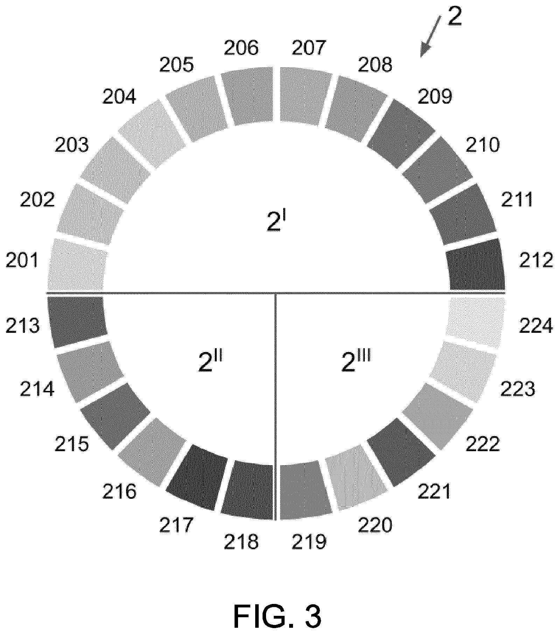

Due to the configuration of the colour roulette 2, the method for standardising an image, based on artificial intelligence algorithms, is able to standardise hue, brightness, saturation, dimension and perspective of an image. The colour roulette 2 comprises the top semi-circumference 2' (Fig. 3) having twelve different pigment values (Fig. 2) and the bottom semi-circumference 2"-2'" (Fig. 3) having another twelve pigment values (Fig. 2). The top semi-circumference 2' having the twelve different pigment values in CMYK scale, placed clockwise, that accurately represent skin-like colours with specific pigment values that match different skin phototypes. The bottom semi-circumference 2"-2'" having the twelve different pigment values that match specific values in both the CMYK and the RGB colour spaces, placed counterclockwise. The twenty four pigment values are:

| Reference Number (Fig. 3) | C | M | Y | K |
|---|---|---|---|---|
| 201 | 0% | 20% | 10% | 0% |
| 202 | 0% | 30% | 20% | 0% |
| 203 | 0% | 30% | 30% | 0% |
| 204 | 0% | 20% | 30% | 0% |
| 205 | 0% | 20% | 30% | 10% |
| 206 | 0% | 30% | 20% | 20% |
| 207 | 0% | 20% | 30% | 20% |
| 208 | 0% | 20% | 30% | 30% |
| 209 | 0% | 30% | 30% | 50% |
| 210 | 0% | 20% | 30% | 50% |
| 211 | 0% | 40% | 40% | 50% |
| 212 | 0% | 40% | 40% | 70% |
| 213 | 0% | 100% | 100% | 0% |
| 214 | 0% | 50% | 100% | 0% |
| 215 | 100% | 0% | 100% | 0% |
| 216 | 50% | 0% | 100% | 0% |
| 217 | 100% | 100% | 0% | 0% |
| 218 | 50% | 100% | 0% | 0% |
| 219 | 100% | 0% | 0% | 0% |
| 220 | 50% | 0% | 0% | 0% |
| 221 | 0% | 100% | 0% | 0% |
| 222 | 0% | 50% | 0% | 0% |
| 223 | 0% | 0% | 100% | 0% |
| 224 | 0% | 0% | 50% | 0% |

Each colour of the colour roulette 2 is separated by white stripes. The colour roulette is externally and internally surrounded by white thin circumferences. Thus, the circle inside the internal circumference is transparent. The same happens outside the external circumference.

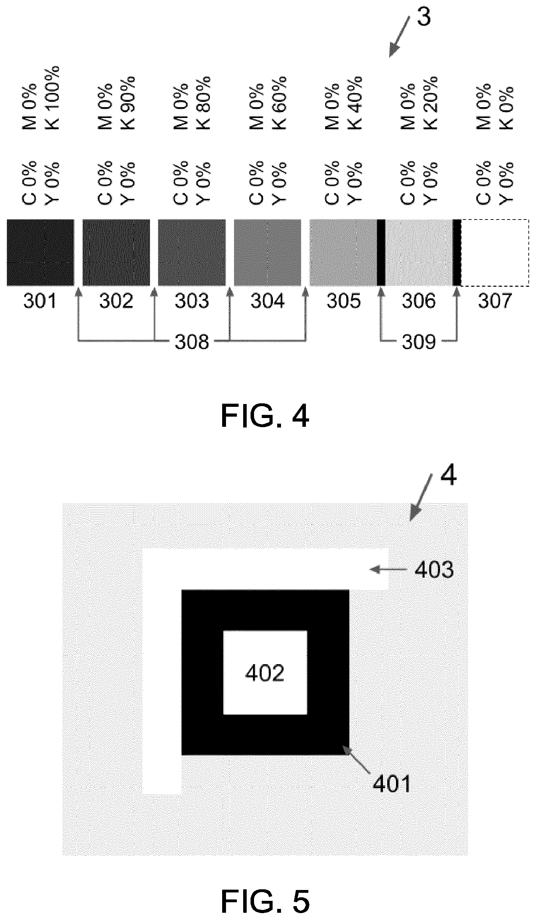

Figure 4 shows the grayscale strip 3 in detail. The grayscale strip 3 is composed of seven squares (301-307) of different pigment values in a row separated by stripes (308,309). The first square 301 is black (CMYK = 0%, 0%, 0%, 100%) and it is placed on the left side of the grayscale strip 3. The seventh square 307 is white (CMYK = 0%, 0%, 0%, 0%) and it is placed on the right side of the grayscale strip 3. The seventh square 307, for the purposes of representing the difference between its white colour and the transparent surface, is delimited by a discontinuous stroke. The second square 302 (CMYK = 0%, 0%, 0%, 90%), the third square 303 (CMYK = 0%, 0%, 0%, 80%), the fourth square 304 (CMYK = 0%, 0%, 0%, 60%), the fifth square 305 (CMYK = 0%, 0%, 0%, 40%), the sixth square 306 (CMYK = 0%, 0%, 0%, 20%) are placed between the first square 301 and the seventh square 307 as shown in Fig. 4. The stripes 308-309 are placed between the squares 301-307, so that the white stripes 308 are placed between the first square 301 to the fifth square 305, and the black stripes 309 are placed between the fifth square 305 to the seventh square 307. These stripes help the artificial intelligence algorithms detect the squares 301-307 with a higher precision.

Figure 5 shows one of the warping elements 4 in detail. The warping element 4 shown in Fig. 5 is the warping element 4 placed on the top left side of the Ai Marker device 1 shown in Fig. 1. The warping element 4 comprises the black square 401 having the white square 402 inside. Both squares 401 and 402 are parallel one to each other. The warping element 4 also has the white "L" bracket 403 externally attached to two sides of the black square 401. The white "L" bracket 403 is delimited by a discontinuous stroke for the purposes of representation, to show the difference between the white colour and the transparent surface. The contrast between the black and white colours improves the performance of the artificial intelligence algorithms comprising the method for standardising an image of the present invention.

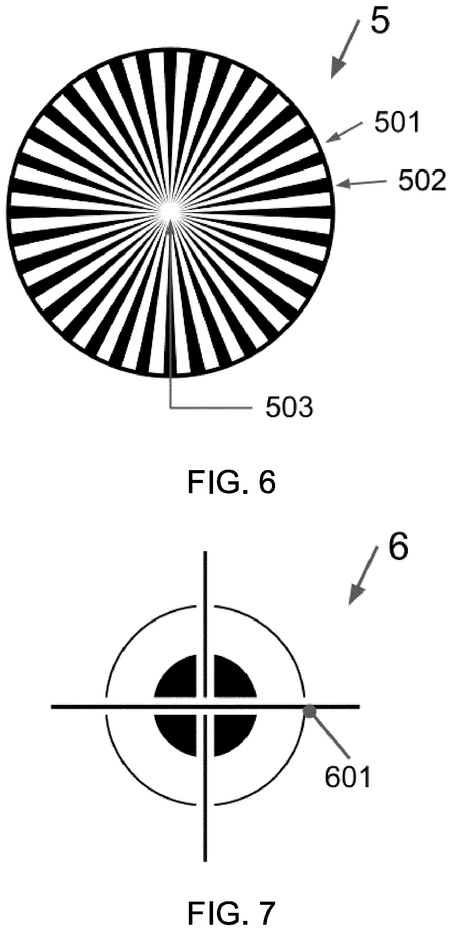

Figure 6 shows the Siemens star 5 known in the prior art. The Siemens star 5 comprises a pattern of bright spokes 501 on a dark background 502 that radiate from a common centre 503 and become wider as they get further from the common centre 503.

Figure 7 shows the registration mark 6 known in the prior art. The registration mark 6 is a black target icon having the centred cross 601, over a transparent surface.

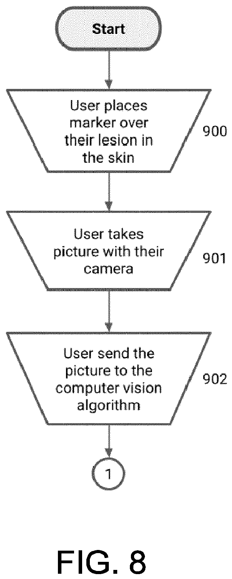

Figure 8 shows a flow chart showing the steps by which a user takes an image of a skin disease having the Ai marker device 1 over it, and sends the image for its processing.

Firstly, the user/patient must put **900** the Ai marker device 1 over the skin disease and take an image **901** with a device provided with a camera, thus generating an image of the skin disease. The image is sent **902** to a computer having means to carry out the method for standardising an image and, optionally also the method for grading the severity of a skin disease.

If it is intended to standardise the captured image using the Ai Marker device 1, it is necessary to carry out the "Ai Marker Detection process", which is a convolutional neural network that performs an object-detection task, in which the category is the whole Ai Marker.

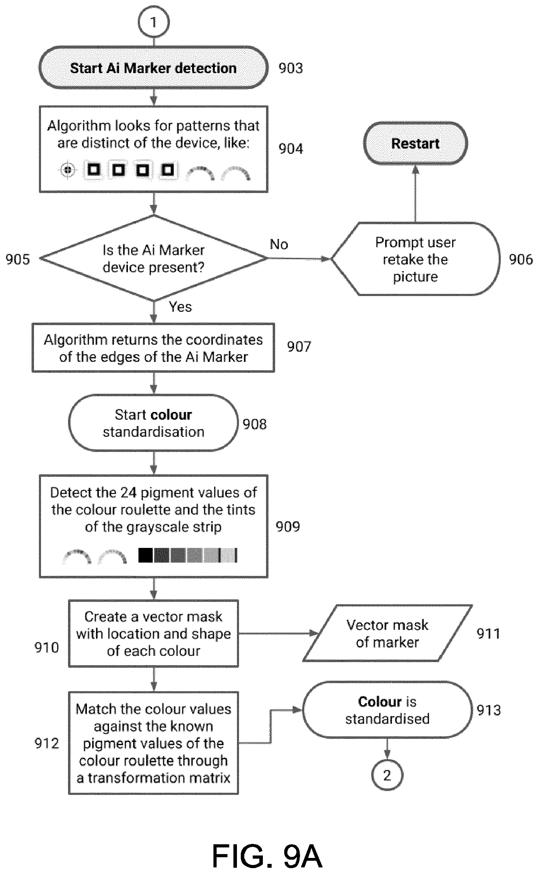

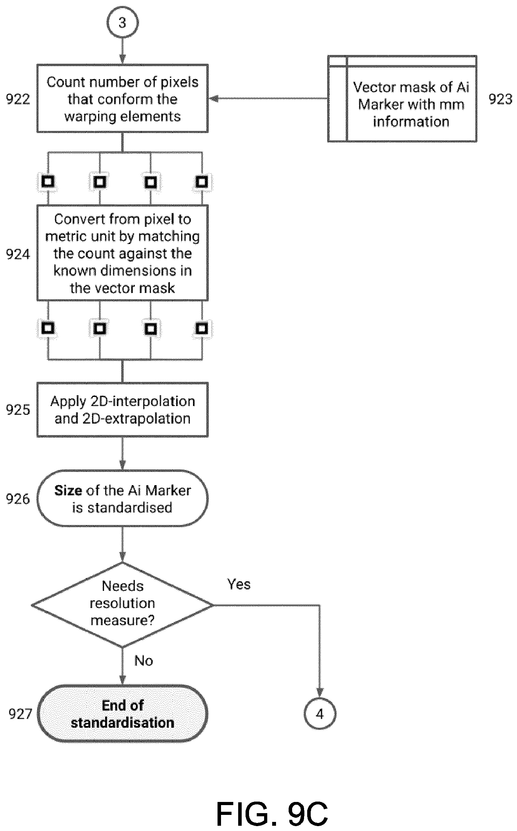

The Ai Marker Detection process finds the Ai Marker device 1 in images, and when the Ai Marker Detection process locates the Ai Marker device, the Ai Marker Detection process gets the coordinates of the Ai Marker device. To do this (FIG. 9A), the Ai Marker Detection process **903** finds **904** the distinctive patterns of the Ai Marker device, such as: the colour roulette 2 (bottom semi-circumference (2", 2"') for hue and top semi-circumference (2') for the Phototype), the grayscale strip 3, the warping elements 4, the Siemens star 5 and the registration mark 6, including the overall arrangement of all the items (2,3,4,5,6). The Ai Marker Detection process returns **907** four coordinates that correspond to the pixels of the image in which the four edges (101, 102 ,103, 104) of the Ai Marker device 1 are placed if the Ai Marker device 1 is included **905** in the image. If the Ai Marker Detection process is not able to return the four coordinates that correspond to the pixels of the image in which the four edges (101, 102 ,103, 104) of the Ai Marker device are placed, it prompts the user to generate another image (retake the picture) **906.** The edges of the Ai Marker device are the two top warping elements of the four warping elements and the first and the seventh squares of the grayscalestrip. The four coordinates are necessary for two things: 1) allows the cropping of the image to automatically exclude pixels out of the region of interest (i.e., pixels surrounding the Ai Marker device), and 2) provides context for the following processes to be applied.

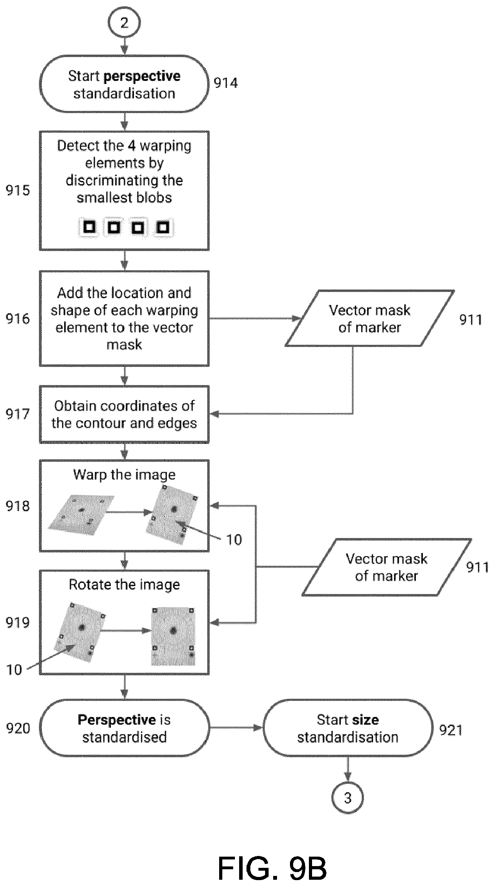

The method for standardising an image using the Ai marker device 1 comprises three further processes: the "Colour standardisation process" **908-913** (FIG. 9A), the "Perspective standardisation process" **914-920** (FIG. 9B) and the "Size standardisation process" **921-926** (FIG. 9B - 9C). The method for standardising may also comprise the "Resolution standardising process" **926-934.**

On each step, a recurring input of the method is the vector mask of the marker **911,** which stores the information about the location and shape of the elements that are detected and calculated along the standardisation method.

The Colour standardisation process **908-913** (FIG. 9A) is a convolutional neural network that performs a segmentation task. The Colour standardisation process detects **909** twenty five pixel categories where each category is one of the 24-colours contained in the bottom semi-circumference 2"-2'" and the top semi-circumference 2', and the remaining category is the non-roulette category. The non-roulette category is what the Colour standardisation process assigns to all pixels in the image that are not part of the colour roulette 2, so they can be discriminated against during the following operations. The Colour standardisation process also creates **910** a mask of vectors **911** that represent the location of each colour in the image.

The goal of the Colour standardisation process is to understand how the colours (pigment values) of the image have been distorted by the lighting conditions of the image capture device. The Colour standardisation process has the task of standardising the colour of the image. To do this, the Colour standardisation process uses the detected twenty four colours (pigment values) and compares them to the known reference values. In other words: the Colour standardisation process gathers the twenty four colours of the colour roulette 2 as they are presented in the image, and gathers the twenty four colours of the colour roulette 2 as they should be presented, in ideal conditions of lightning. Then, the Colour standardisation process performs **912** a transformation matrix to the rest of the image based on the vector mask.

The Colour standardisation process returns **913** a standardised image as a result of the transformation matrix. The transformation matrix is used to transform the colour space based on the vector mask of the whole image into the standard colour space. This can be understood as a "colour standardising", and enables the Colour standardisation process to turn any two images, taken under different lighting conditions, into perfectly comparable images regarding their colour space.

The Perspective standardisation process **914-920** (FIG. 9B) is a convolutional neural network that performs a segmentation task. The perspective standardisation process detects nine pixel categories: two of them belong to each colour of the Warping elements 4: the white and the black; and the remaining seven categories correspond to each of the tints in the grayscale strip 3. The goal of the perspective standardisation process is to understand how the Ai Marker device 1 is oriented and how it has been distorted by the perspective of an image capture device (like a Smartphone).

The perspective standardisation process **914** firstly detects **915** the four warping elements **4.** In this process, to reduce error, the Perspective standardisation process discriminates the smallest blobs detected and keeps the four biggest ones, to make sure that only the right Warping elements **4** have been detected. Then, it adds the location and shape of each warping element to the vector mask **916** that was previously created (911). Thanks to this, the Perspective standardisation process calculates **917** the contour and the coordinates of the edges of the warping elements **4.** Then, the Perspective standardisation process warps **918** the image using the coordinates of the warping elements so that the plane of the Ai Marker device is perfectly perpendicular, and thus, the ratio of the Ai marker device is 1:1.314. Then, the perspective standardisation process uses the information of the vector mask 911 that contains the coordinates of the edges of the grayscale strip and the warping elements. After that, the Perspective standardisation process rotates **919** the image so that the grayscale strip 3 is always rotated zero degrees, in the bottom part of the image 10. This allows to make any two images comparable regarding their perspective. For instance, if a doctor takes images of a melanoma a week apart, and the perspective of the images was different, it is difficult to see if the lesion has grown, because it is distorted by the perspective. But after the Perspective standardisation process has warped the images so that they are standardised images **918** perfectly perpendicular and straightened **919,** it is easy to compare them. It is also possible to put one above the other, with decreased opacity, and clearly see how the edges of the pathology have changed.

The Size standardisation process **921-926** (FIG. 9B - 9C) uses the detected pixels of the Warping elements 4 that have been provided by previous processes and stored in the vector mask. The process calculates **922** the area of the warping elements in square pixels by counting the number of pixels that comprise the warping elements 4 in the vector mask **923..** Since the Size standardisation process already knows the actual area of the Warping elements 4 in millimetres, it converts **924** the pixels of each warping element to metric units values matching the area in square pixels against the known dimensions in square millimetres. Then, the Size standardisation process performs **925** a 2D-interpolation and a 2D-extrapolation by means of conversion between pixels and the known metric units on each of the four Warping elements 4. Thus, the Size standardisation process is able to calculate the value in metric units of each and every single pixel of the image. This way, the Size standardisation process can measure the surface (in metric units) of any skin disease of an image, as long as the skin diseases are contained inside the Ai Marker device 1. The Size standardisation process finally generates a standardised image in size **926** and also in colour and perspective **927.**

However, the Size standardisation process can also perform this operation to measure skin pathologies that are outside of the Ai Marker device 1. This is useful in case the area affected by the pathology is larger than the Ai Marker device 1. Then, to calculate the conversion in any other point of the image, the Size standardisation process gathers the pixel-to-metric unit conversion of each warping element 4, and performs a 2D-extrapolation and a 2D-interpolation **925** that allows it to measure the metric unit size of each and every single pixel of the image, regardless its relative position towards the Ai Marker device 1. This way, the Size standardisation process knows exactly the surface of any skin disease in millimetres, even if it is outside the Ai Marker device 1.

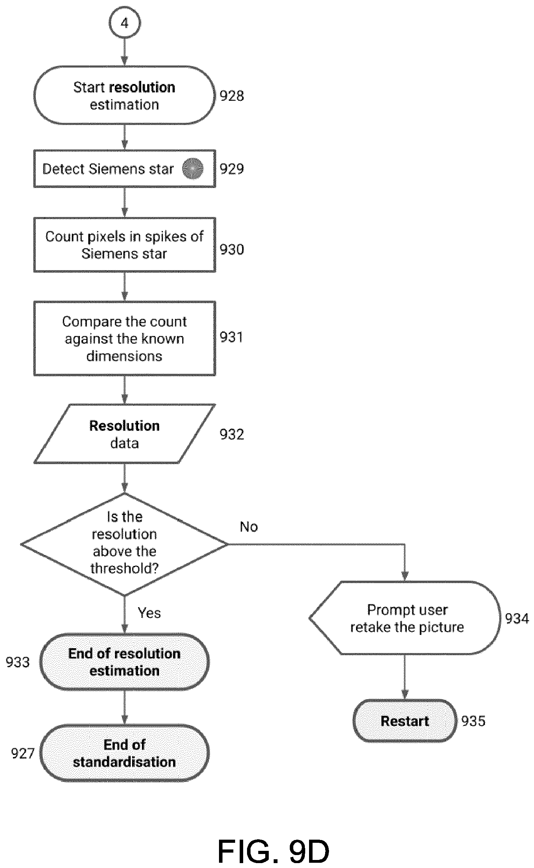

Additionally, there is one extra process that is completely optional (FIG. 9D), as it depends on the goals of the user: the "Resolution standardising process" **928-934.** If a resolution measurement is needed, the Resolution standardising process is applied to the image taken by the user.

The Resolution standardising process starts by **928** (FIG. 9D) detecting **929** the Siemens star 5. Then, the resolution standardising process calculates resolution and information about the resolution by counting **930** how many pixels constitute the spikes 502 of the Siemens star 5. Then, the process compares **931** the count against the known dimensions of the Siemens star and returns **932** a value that reflects the resolution of the image. If the resolution is above a threshold, the resolution standardising process is over **933** and the method for standardising an image is also over **927.** If not, the Resolution standardising process prompts the user to retake the image **934** restarting **935** the whole process from the beginning (Fig. 8, 900).

The Resolution standardising process can be run at different points of the method for standardising an image. For instance: it can be useful to automatically discard images, specifically when managing large datasets, before sending them to the processing cue. It can also be used in every step of the process, as a countermeasure to learn if the Perspective standardisation process or the Colour standardisation process has decreased the resolution of the image. And it can be useful also at the end, as an automatic quality assurance process.

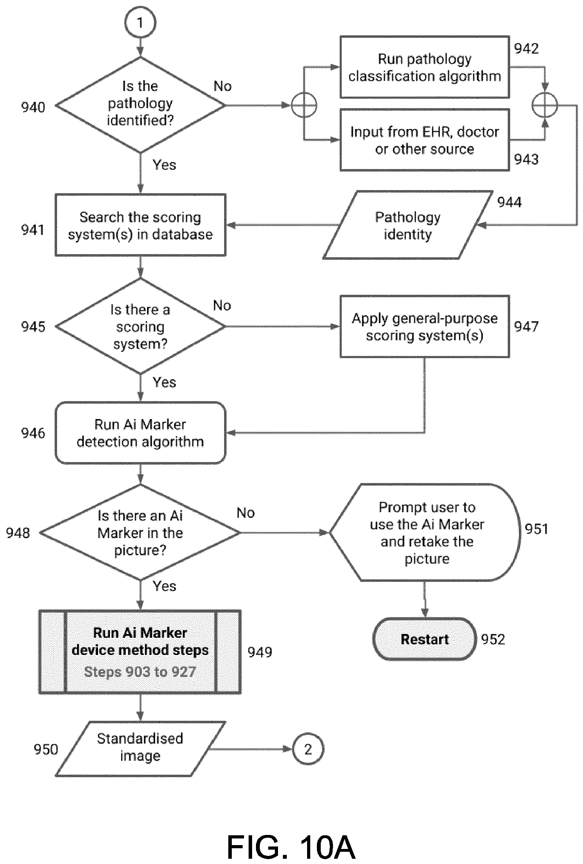

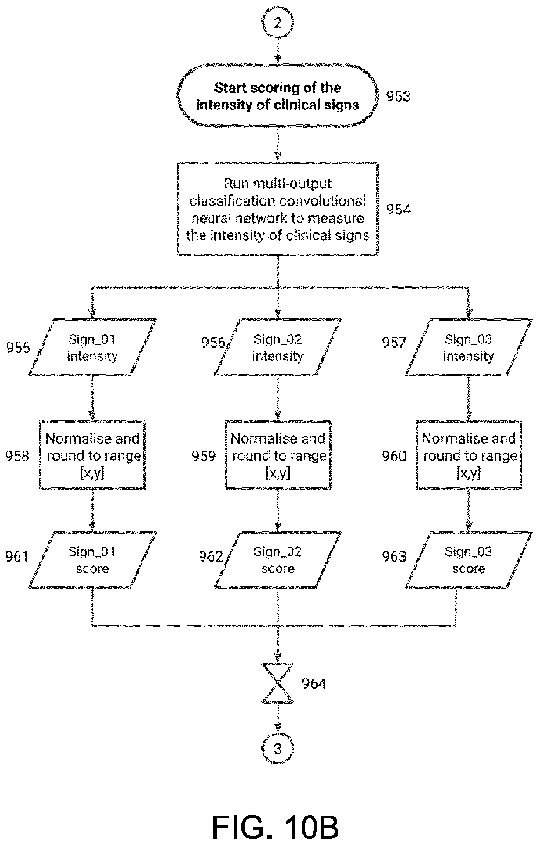

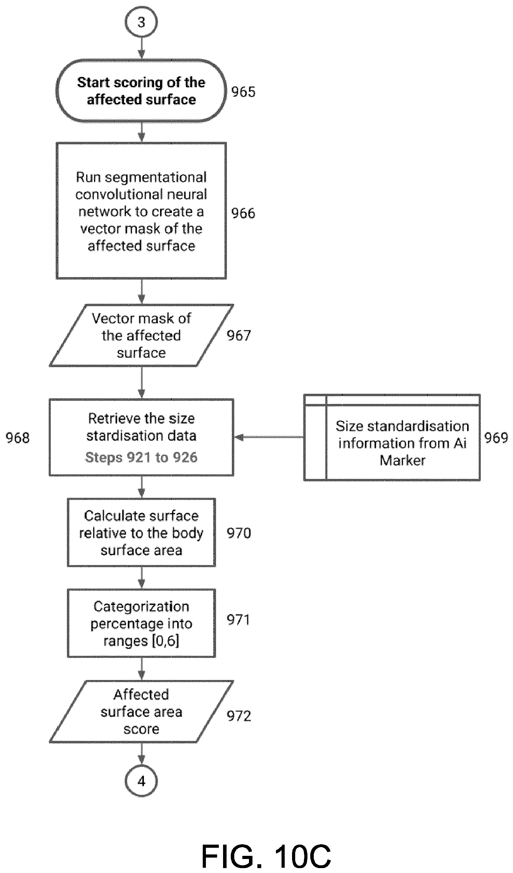

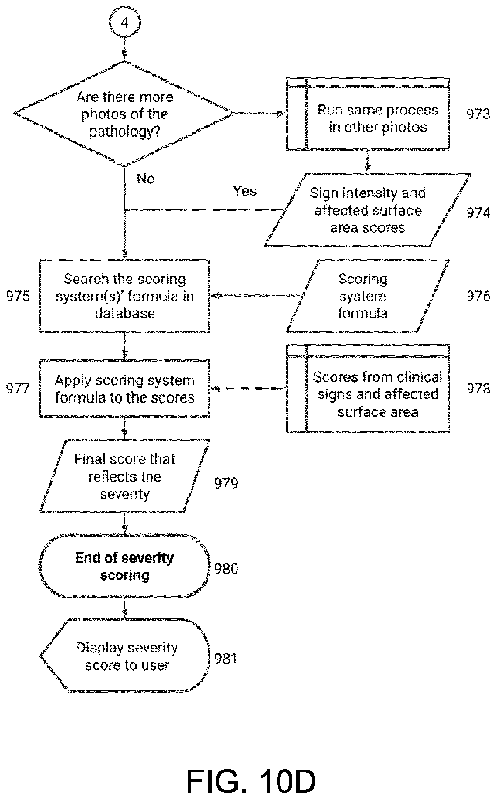

Figures 10A, 10B, 10C and 10D show a method for grading a skin disease using the Ai marker device and the computer-implemented method for standardising an image of the present invention. This method can be applied to any other disease. For this particular disease, i.e., the "skin" disease, the top semi-circumference (2') consists of twelve skin-like colours with specific pigment values that match different skin phototypes. In case of any other disease, the pigment values of the top semi-circumference (2') have to be particularised.

The method for grading a skin disease using the Ai marker device and the computer-implemented method for standardising an image of the present invention may start **940** by trying to identify the pathology. This is not essential for the scoring of the intensity of clinical signs **953.** If the pathology is not known, the method for grading a skin disease runs **942** the "Pathology classification" process or it uses **943** another input like an Electronic Health Record (EHR) software, a doctor or any other source.

Once the pathology is identified **944,** the present invention may gather the information about which scoring system is applicable for the identified pathology **941** in a database. This is also an optional step. If the scoring system is not found **945,** a general-purpose scoring system may be applied **947.**

The next step is to standardise the image. This step is needed for an optimal performance of the invention. For this, the invention runs **946** the "Ai Marker detection process", which is able to find **948** the Ai Marker device in a picture/image. If the Ai Marker detection process returns that the picture does not contain the Ai Marker device, the user is prompted **951** to use the Ai Marker device and retake the picture. If the Ai Marker detection process turns out that the Ai Marker device is included in the taken picture, previous steps 903 to 927 are carried out **949** to get the image standardised **950.**

Once the image is standardised, it starts **953** the scoring of the intensity of clinical signs, consisting of running **954** a multi-output classification Convolutional Neural Network "CNN" to measure the intensity of clinical signs. The clinical signs of skin disease are redness, swelling, oozing, scratch marks, lichenification, dryness, induration or desquamation, to name a few. For each clinical sign, the multi-output classification convolutional neural network calculates **955-956-957** the intensity. Then, each sign is normalised and rounded to a range [x,y] **958-959-960** to finally be scored **961-962-963.**

The next step is to score the affected surface **965,** consisting of calculating the affected surface by running **966** a segmentational convolutional neural network to create a vector mask of the affected surface **967.**

Then, the previous steps 921-926 for size standardisation with the Ai Marker device are applied **968** to the vector mask of the affected surface using **969** the size standardisation information from the Ai Marker. Once the size is standardised and it knows the real dimensions of the image, the method for grading a skin disease calculates the surface of the lesion relative to the body surface area **970.** After that, a categorization percentage into ranges is calculated **971.** For instance, the ranges can be from "0" to "6" as shown in Fig. 10C. As a result the affected surface area is scored **972.**

In an arrangement, the method may further comprise (FIG. 10D) to search the scoring system's formula in a database **975** in order to apply a specific formula that computes the scores returned in steps **964** and **972.** For instance, the "PASI" (Psoriasis Area and Severity Index) scoring system for psoriasis will ponderate the intensity of erythema, desquamation and dryness to return a score from "0" to "72" that reflects the severity of psoriatic lesions. This step is optional.

In order to calculate the evolution of the pathology, the method for grading a skin disease further comprises to run **973** the previous steps **940-972** to other pictures/images of the same affected surface area, if any, turning out the sign intensity and the affected surface areas score for each picture/image **974.**

Then, the resultant intensity of the clinical signs and the affected surface area for each picture/image may be introduced in the scoring system formula **976** in order to calculate **978** the severity score. The scores from the clinical signs and the affected surface area are used in the scoring system's formula **977** to obtain the "Final score" **979** that reflects the severity, ending **980** the method for grading a skin disease severity, i.e., the severity scoring, which can be displayed **981** to a user.

## Claims

1. An Ai marker device (1) for standardising an image, which comprises:
- a colour roulette (2) configured to standardise hue, brightness, saturation, dimension and perspective of an image (10); the colour roulette (2) comprising at least:
∘ a bottom semi-circumference (2^{I}, 2") comprising at least six colours (2^{III}) with specific pigment values, wherein the six colours having three CMY pure pigment values and three CMY pure pigment values at 50%;
- a grayscale strip (3) under the colour roulette configured to standardise brightness of the image, the grayscale strip comprises a gradient of tints of black;
- four warping elements (4) forming vertices of a square in which the colour roulette is circumscribed, the four warping elements (4) configured to correct a perspective of the image;
- a Siemens star (5) placed between the grayscale strip and the colour roulette configured to calculate resolution of the image;
- a registration mark (6) configured to detect the Ai marker at first sight;
the colour roulette (2), the grayscale strip (3), the four warping elements (4), the Siemens star (5) and the registration mark (6) be placed on a transparent surface (7), which inside the colour roulette (2) is cut-out.

2. The Ai marker device for standardising an image of claim 1, wherein the bottom semi-circumference (2", 2^{III}) further comprises three further colours (2") having three RGB pure pigment values and three more colours (2") that are exact combinations of the RGB pure pigment values.

3. The Ai marker device for standardising an image of claim 1, wherein the colour roulette (2) further comprises a top semi-circumference (2^{I}) consisting of twelve skin-like colours with specific pigment values that match different skin phototypes.

4. The Ai marker device for standardising an image of claim 1, wherein the transparent surface (7) is manufactured in a transparent plastic film and laminate polyethylene transparent paper, or be manufactured in polypropylene.

5. The Ai marker device for standardising an image of claim 3, wherein the top semi-circumference having the following twelve different pigment values in CMYK scale, placed clockwise:
| C | M | Y | K |
|---|---|---|---|
| 0% | 20% | 10% | 0% |
| 0% | 30% | 20% | 0% |
| 0% | 30% | 30% | 0% |
| 0% | 20% | 30% | 0% |
| 0% | 20% | 30% | 10% |
| 0% | 30% | 20% | 20% |
| 0% | 20% | 30% | 20% |
| 0% | 20% | 30% | 30% |
| 0% | 30% | 30% | 50% |
| 0% | 20% | 30% | 50% |
| 0% | 40% | 40% | 50% |
| 0% | 40% | 40% | 70% |

6. The Ai marker device for standardising an image of claims 1 to 3, wherein the bottom semi-circumference having the following twelve different pigment values in CMYK scale, placed counterclockwise:
| C | M | Y | K |
|---|---|---|---|
| 0% | 100% | 100% | 0% |
| 0% | 50% | 100% | 0% |
| 100% | 0% | 100% | 0% |
| 50% | 0% | 100% | 0% |
| 100% | 100% | 0% | 0% |
| 50% | 100% | 0% | 0% |
| 100% | 0% | 0% | 0% |
| 50% | 0% | 0% | 0% |
| 0% | 100% | 0% | 0% |
| 0% | 50% | 0% | 0% |
| 0% | 0% | 100% | 0% |
| 0% | 0% | 50% | 0% |

7. The Ai marker device for standardising an image of claim 1, wherein the grayscale strip (3) comprises seven squares, a first square (301), a second square (302), a third square (303), a fourth square (304), a fifth square (305), a sixth square (305) and a seventh square (307), in a row separated by stripes (308,309); wherein the first square is black and the seventh square is white, and the second square, the third square, the fourth square, the fifth square and the sixth square being grey from dark grey to light grey, respectively; and wherein the stripes (308,309) between the first square to fifth square are white stripes (308) and the stripes between the fifth square to the seventh square are black stripes (309).

8. The Ai marker device for standardising an image of claim 7, wherein the first square (301) having a CMYK equal to 0%, 0%, 0%, 100%, the second square (302) having a CMYK equal to 0%, 0%, 0%, 90%, the third square (303) having a CMYK equal to 0%, 0%, 0%, 80%, the fourth square (304) having a CMYK equal to 0%, 0%, 0%, 60%, the fifth square (305) having a CMYK equal to 0%, 0%, 0%, 40%, the sixth square (306) having a CMYK equal to 0%, 0%, 0%, 20%, and the seventh square (307) having a CMYK equal to 0%, 0%, 0%, 0%.

9. The Ai marker device for standardising an image of claim 1, wherein each warping element (4) comprises a black square (401) having a white square (402) inside, being parallel one to each other; and wherein each warping element (4) comprises a white "L" bracket (403) externally attached to two sides of the black square (401).

10. The Ai marker device for standardising an image of claim 1, wherein the Siemens star (5) comprises a pattern of bright spokes (501) on a dark background (502) that radiate from a common centre (503) and become wider as the bright spokes (501) get further from the common centre (503).

11. The Ai marker device for standardising an image of claim 1, wherein the registration mark (6) is a black and white target icon having a centred cross.

12. A computer-implemented method for standardising an image using an Ai marker device according to any one of claims 1 to 11, wherein the method comprises:
a) starting (903) the Ai Marker detection process, comprising:
∘ locating (904) an Ai marker device in an image by finding the colour roulette, the four warping elements, the grayscale strip, the Siemens star and the registration mark of the Ai marker device; if the Ai marker device is not found (905), prompt (906) the user to retake the image; if it is found, continue;
∘ calculating (907) four coordinates of pixels of the image in which edges of the Ai Marker device are placed;
b) starting (908) the colour standardisation process, comprising:
∘ detecting (909) pigment values of the colour roulette and the grayscale strip in the image;
∘ creating (910) a mask of vectors (911) that contains the location and shape of each colour of the roulette and the grayscale strip; and,
∘ performing (912) a transformation matrix to standardise the hue and saturation, by matching the colour values as they are captured in the image against the known pigment values of the colour roulette;
c) starting (914) the perspective standardisation process, comprising:
∘ detecting (915) the four warping elements;
∘ adding (916) the location and shape of each warping element to the vector mask (911);
∘ obtaining (917) the coordinates of the contour and the coordinates of the edges of the four warping elements and the grayscale strip;
∘ correcting (918) the perspective by means warping the image so that the plane is perfectly perpendicular; and,
∘ correcting (919) the perspective by means of rotating the image;
d) starting (921) the size standardisation process, comprising:
∘ calculating (922) the area of the warping elements in square pixels by counting the number of pixels that conform the warping elements in the vector mask (923);
∘ converting (924) the pixels of each warping element to metric units values matching the area in square pixels against the known dimensions in square millimetres;
∘ performing (925) a 2D-interpolation and 2D-extrapolation to calculate the value in metric units of each and every single pixel of the image; and,
∘ generating (927) a standardised image in size, colour and perspective.

13. The computer-implemented method for standardising an image of claim 12, wherein the method further comprises starting (928) the resolution standardising process, comprising:
∘ looking (929) for a Siemens star (5);
∘ calculating resolution and information about the resolution by:
▪ counting (930) how many pixels constitute the spikes (502) of the Siemens star (5);
▪ comparing (931) the count against the known dimensions;
▪ returning (932) a value that reflects the resolution of the image.

14. A method for grading the severity of a skin disease, comprising;
a) providing an Ai marker device according to any one of claims 1 to 11;
b) placing the Ai marker device in the vicinity of a skin disease of a patient and taking an image of the skin with the Ai marker device;
c) applying a computer-implemented method for standardising an image according to claim 12, that returns (950) the standardised image;
d) starting (953) the scoring of the intensity of the clinical signs, comprising:
∘ for each clinical sign, running (954) a multi-output classification convolutional neural network that returns (955-957) the intensity of each clinical sign;
∘ normalising and rounding (958-960) to a range the intensity of the clinical signs that returns the score of the clinical signs (961-963);
∘ aggregating (964) all the scores.
e) starting (965) the scoring of the affected surface, comprising:
∘ running (966) a segmentational convolutional neural network, that returns (967) a vector mask of the affected surface;
∘ retrieving (968) the standardised image (969) that was calculated on step c) and enables the standardisation of the size of the image;
∘ calculating (970) the surface of the lesion contained in the image relative to the whole body surface area;
∘ categorising (971) the percentage of the affected body area into ranges, which constitutes (972) the score of the affected surface area.

15. The method for grading the severity of a skin disease of claim 14, further comprising;
f) if the user is taking more than one image of the same patient, running (973) previous steps a) to e) and applying them to further pictures/images, which return (974) the scores of the intensity of the clinical signs and the score of the affected surface area for each image;
g) retrieving (975) the scoring system's formula (976) in a database of medical scoring systems;
h) applying (977) the scoring system's formula (976) to the scores of the intensity of the clinical signs and the score of the affected surface area (974), that returns (980) a final score that reflects the severity of the disease; and,
i) displaying (981) the final score.
